# EUROPEAN PATENT SPECIFICATION

(11) **EP 1 362 518 B1**
(45) Date of publication and mention of the grant of the patent: **27.06.2007**
(21) Application number: 02076890.9
(22) Date of filing: 15.05.2002
(51) Int. Cl.: A23L 1/10, A61K 31/205, A23L 1/30, A23L 1/308, A23L 1/302, A23L 1/305, A23L 2/52

(54) **Food products providing sustained blood levels of caffeine and their use**
Lebensmittel für konstante Blutspiegel von Koffein und ihre Verwendung
Aliments fournissant des niveaux sanguins constants de caféine et leur utilisation

(43) Date of publication of application: 19.11.2003
(73) Proprietor: NESTEC S.A., 1800 Vevey (CH)
(72) Inventor: Decombaz, Jacques, CH-1806 St-Legier (CH); Milon, Hubert, CH-1053 Cugy (CH); Wuersch, Pierre, CH-1814 La Tour-de-Peilz (CH)
(74) Representative: Dixon, Sarah

(56) References cited:
- EP-A- 0 524 484
- EP-A- 0 554 008
- EP-A- 1 048 690
- WO-A-01/03691
- WO-A-01/41795
- WO-A-01/84950
- WO-A-93/06737
- DE-U- 29 805 782
- US-A- 4 871 557
- US-A- 5 932 561
- DATABASE WPI Section Ch, Week 200175 Derwent Publications Ltd., London, GB; Class B05, AN 2001-650391 XP002218590 & JP 2001 169753 A (TAKEDA SHOKUHIN KOGYO KK), 26 June 2001 (2001-06-26)
- DATABASE WPI Section Ch, Week 200114 Derwent Publications Ltd., London, GB; Class D13, AN 2001-128231 XP002218591 & JP 2000 316478 A (BIO ARUBIN KENKYUSHO KK), 21 November 2000 (2000-11-21)
- PATENT ABSTRACTS OF JAPAN vol. 1995, no. 09, 31 October 1995 (1995-10-31) & JP 07 147919 A (UNIE KAFUE:KK), 13 June 1995 (1995-06-13)

## Description

The present invention relates to the use of a mixture comprising caffeine and a soluble fibre in the preparation of a food product to avoid short-term peaks of caffeine in serum. It further relates to a food product comprising soluble fibre and caffeine..

The invention further relates to a consumable package comprising a liquid and a solid product and the use of the liquid and the solid product in the preparation of a consumable package for rapidly achieving a high serum concentration of caffeine and for sustaining this high concentration for a prolonged time.

### The Background Art

The temporal distribution of substances appearing in body fluids of humans and animals due to digestion and absorption of food is difficult to control. The concentration of some of these substances in blood, for example glucose, can be regulated by the body in healthy individuals, within a certain range. This regulation guarantees a constant and sufficient supply of glucose to all parts of the body. The concentration of glucose in blood is carefully controlled by the concerted action of different organs, including the release of hormones with specific activities. If its concentration in blood tends to become high after a meal, glucose will be stored in the liver or in muscles in a polymerised form as glycogen. On the other hand, if the concentration of glucose in blood decreases, it is released from the liver into the blood stream, to the final end that a more or less constant glucose level in blood is achieved and homeostasis in the entire body can be upheld. As with glucose, some sort of a biochemical mechanism to control the concentration in blood exists also for other substances, for example lipids etc.

However, the human or animal body is devoid of a regulatory system for many other substances that enter the body through the gastro-intestinal tract. Many such substances cannot be stored or re-synthesised. Of course, these substances include an endless variety of chemical classes, and they may (or not) perform a likewise huge variety of functions or effects in the body. Some of them are essential substances, for example vitamins. If these substances are not consumed on a regular basis, the body reacts by symptoms of lack. Other exogenous substances are administered orally in order to achieve a specific pharmacological effect. For example, fluoxetine, a substance that inhibits serotonin-uptake activity in the synapses of humans, is administered in order to alleviate pain. Still other exogenous substances have less pronounced or no physiological effects.

Another example is caffeine, a mild central nervous system stimulant naturally occurring in over 60 plant species in various parts of the world, such as the coffee-bean and the tea-leave, in the seeds of the cocoa plant and in the guarana root.

If orally consumed, caffeine will be absorbed from the gastro-intestinal tract and reach the circulation of the blood. Its level in blood will be directly proportional to the amount of what was consumed. For example, if a cup of coffee is consumed, usually after about an hour a peak in caffeine level in blood will occur, which correlates with the amount that has been consumed. The body eliminates caffeine by hepatic biotransformation (most of the caffeine is metabolized to paraxanthine in the liver) and renal excretion of caffeine or metabolites. The half-life of caffeine in adults is about 3-7 hours. As becomes evident, the concentration of an exogenous substance as, for example, caffeine, in blood over time is characterized by a quick increase of the substance shortly after consumption and a relatively slow disappearance after this peak. Hence, the body has not the ability, as is the case with glucose, to uphold a constant concentration of the substance in blood over a longer period of time.

A lot of specific problems are tightly linked to the above, general problem. Again caffeine may serve as an example. It has a number of effects that are tightly related to the blood concentration. For example, caffeine has psychostimulant properties, evidently due to its effects on all parts of the central nervous system. Besides this, it increases respiratory rate, heart rate, blood pressure and secretion of stress- and other hormones. Caffeine is therefore used, in adults, as a mild central nervous system stimulant that helps to restore mental alertness or wakefulness when fatigue or drowsiness is experienced. These effects even extend to happiness, a good mood or other positive experiences, which accounts for the taxation of caffeine as a psychostimulant. In larger doses, it stimulates medullary, vagal, vasomotor and respiratory centres. In still larger doses, side effects of caffeine appear like dizziness, fast heartbeat, nervousness, troubles in sleeping. Other symptoms of an overdose are abdominal or stomach pain, agitation, anxiety, muscle trembling. Trials with dogs report that even death can occur, if 100mg/kg/d (caffeine/body weight/day) is consumed for more than 3 days. Although this list of beneficial and adverse effects of caffeine is by no means exhaustive, it becomes evident that there is an ideal or desired range of concentration of caffeine in blood. For caffeine, the threshold value for stimulation is at about 2 mg/l blood plasma, whereas the upper end of a the desired range may be situated at about 20 mg/l blood plasma. When, for example, a cup of coffee is consumed, a peak in blood-caffeine level is reached after one hour, entailing the corresponding effects. After a short period of about 3 hours, the effects of this exogenous substance decline and stop, because the threshold value of 2 mg/l blood plasma is fallen below. If another cup of coffee is consumed, however, not only will the threshold value soon be exceeded, but even first side effects may turn up, because the caffeine concentration has crossed the upper level of the ideal range. The peak in caffeine blood concentration of the second (or a further) cup may lead symptoms like trembling, nausea, gastro-intestinal irritation, frequent urination, headache, asthenia and so forth.

Therefore, a need exists for administering or supplying caffeine in a way that its concentration in the blood of the consumer remains constant over a prolonged period of time.

There is also a need for administration of caffeine in a way that prevents a "peak", that is, a transiently too high concentration of the substance in blood. This is even more of a problem, if side effects have to be expected at high concentrations.

Furthermore, there is a need for consumable products that sustain an adequate concentration of a selected substance in blood in the range of its effectivity. In other words, the blood concentration need not only be maintained, but maintained at a useful concentration.

Of course, if a mechanism is found that is suitable to alleviate the problems as set forth above, this mechanism must have no negative impact on the consumer and, furthermore, it preferably shouldn't interact with the caffeine. It would be an advantage, if a mechanism addressing these problems was based on natural substances the tolerance of which is not an issue and has not to be proven.

There is a general need for food products, including beverages that confer caffeine for a sustained period.

More precisely, there is a need to prevent an unacceptably high peak of caffeine in blood after consumption of a caffeine-containing food-product or beverage. However, the need also demands a concentration of caffeine that is for a prolonged time in the range of stimulation.

It is widely known that the absorption of drugs in the digestive tract is decelerated, diminished or, contrarily, accelerated if the drug is taken after or together with a meal. However, in this case, a drug may not be consumed at any desired time, but only after a meal. Furthermore, the components of the meal may vary strongly in a way that no prediction concerning the appearance of the drug in blood may be made.

Moreover, to date, if it is wished to administer a substance that is pharmalogically active in a way that it appears constantly in blood, this substance may be injected by infusion over several hours. Infusions, however, entail a number of disadvantages that may be avoided.

In DE 19841209 (GOENING R) caffeine tablets produced by direct compression of caffeine-containing plant materials or extracts are disclosed. Sucking the tablets provides a slow release of caffeine in the gastrointestinal tract. However, the tablets are usually consumed over a short time (5 minutes, for example), and the caffeine liberated within that time will appear quickly in blood.

In FR2345938 (CHOAY P) a chewing gum containing xanthine stimulants or mixtures thereof, including plant extracts e.g. tea and coffee extracts is disclosed. The chewing gum also contains an absorbant for a slow release of the stimulant, such as silicic acid and cationic exchange resins. As an advantage, the effects of the stimulants persist after the user discharged it. However, the administration of caffeine in this case demands a prolonged time of chewing.

Various patents disclose different formulations containing soluble fibre and caffeine. For example, JP-A-2001 169 753 discloses a composition containing, inter alia, a xanthine derivative such as caffeine and a dietary fibre such as pectin or guar gum which is stated to be effective in the control of blood cholesterol levels. JP-A-2000 316 478 discloses a functional coffee beverage which contains dextrin and is stated to inhibit increases in post-prandial blood glucose levels. JP-A-07 147 919 discloses a coffee gruel containing coffee, milk and a digestible carbohydrate such as rice. This gruel provides a coffee flavoured drink consumption of which also provides the macronutrients protein, fat and carbohydrate. EP 554 008 discloses a coffee beverage which contains soluble fibre derived from coffee beans and a process for obtaining such fibre from coffee beans whilst EP 524 484 discloses an instant beverage powder containing a concentrate of tea or tea substitutes on a substrate which comprises inulin. However, none of these prior documents relates to sustained delivery of caffeine over an extended period.

The present invention addresses the problems set out above.

### Summary of the Invention

The present invention is based on the surprising discovery that soluble fibre, if consumed together with caffeine, is able to slow down release and absorption of caffeine from the digestive tract in a way that its concentration in serum remains above a given value for a prolonged time.

Remarkably, it was also found that by combining a solid food product with a beverage, both of them comprising caffeine, the solid product further comprising soluble fibre, a high concentration of caffeine in serum is quickly achieved and sustained for a prolonged period.

Consequently, in a first aspect the present invention provides the non-therapeutic use of a mixture of caffeine and soluble fibre in sufficient amounts in a food product to avoid short term peaks of the caffeine in plasma and/or to keep the plasma concentration of caffeine maintained for a prolonged period. The invention further extends to the use of a mixture of caffeine and soluble fibre in sufficient amounts in the manufacture of a therapeutic food product to avoid undesired side effects of short team peaks of caffeine in plasma.

In a second aspect, the present invention provides the non-therapeutic use of a mixture of caffeine and soluble fibre in sufficient amounts in a food product to slow down the absorption rate of the caffeine in the gastrointestinal tract.

In a third aspect, the present invention provides a food product comprising from 2 to 40% by weight of viscous soluble fibre and from 200 to 400 mg caffeine.

In a fourth aspect, the present invention provides a consumable package comprising a liquid and a solid product, wherein the solid product is the food product according to any of claims 7 to 9 and the liquid product also comprises caffeine.

An advantage of the present invention is that it prevents a high and short-term peak of the concentration of caffeine in blood.

Another advantage of the present invention is that a high peak entailing undesired side effects of caffeine is avoided.

Still another advantage of the present invention is that it provides a sustained concentration of caffeine in blood.

Yet another advantage of the present invention is that it provides a concentration of caffeine in serum lying in the optimal range of effectiveness of the substance.

Another advantage is, that many soluble fibres are known as food-components for a long time and may therefore be used without testing tolerance in humans or animals.

An big advantage is provided by the fact that soluble fibre has a beneficial effect on the consumer. For example, it may be a prebiotic and/or it may provide energy for a prolonged time, if glucose is also present in a food product. Hence, two very different effects are achieved by using soluble fibre explicitly in a food product.

Furthermore, it is an advantage that soluble fibre need not be isolated in order exert the desired effect as described above. Natural products that contain soluble fibre, for example vegetables and cereals, for example oat and barley and their bran, for example, are easily available and may directly be used.

In the figures,

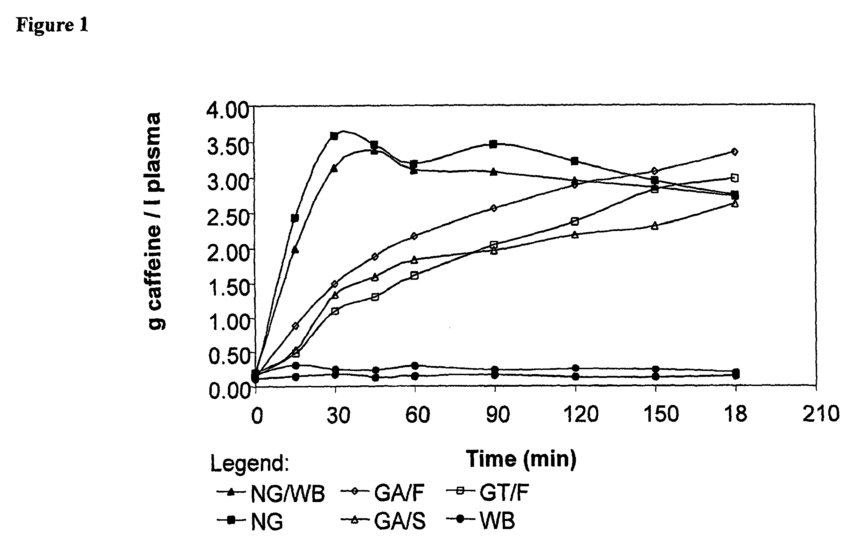

Figure 1 shows caffeine plasma levels over time (3 hours) in human individuals that consumed caffeine with and without soluble fibre. Caffeine was consumed in the form of a cup of soluble coffee (NG), a coffee together with white bread (NG/WB), in a bar comprising oat bran and green tea (OBC-GT), oat bran and Guarana powder (OBC-Gu), and, oat bran and Guarana, where the latter was added to the syrup in the preparation of the bar (OBC-GS). Oat bran is rich in soluble fibre (β-Glucan).

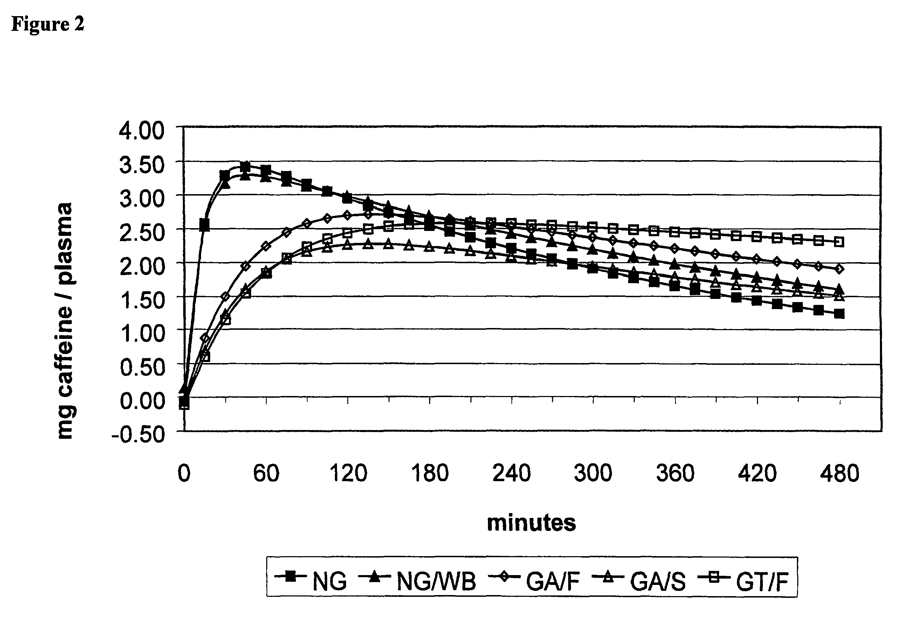

Figure 2 shows modelised caffeine plasma levels over a prolonged time (8 hours) based on caffeine plasma and salivary levels in human individuals that consumed caffeine with and without soluble fibre. The values were fitted by calculation to levels that would appear in blood. Details are given in Example 2. The same symbols were used as in Figure 1.

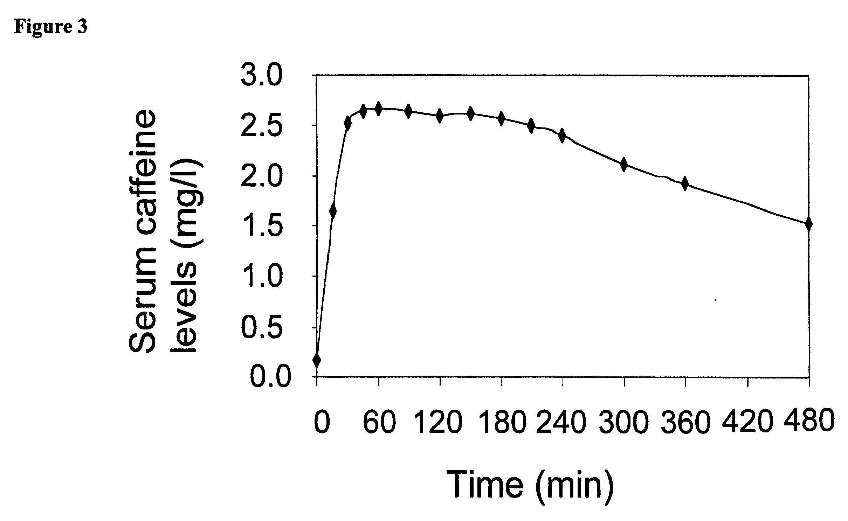

Figure 3 represents a simulation of the simultaneous consumption of a cup of coffee and a bar, each comprising 100mg caffeine, the bar further comprising soluble fibre. It can be seen that a high serum concentration of caffeine is obtained quickly and that the high concentration is sustained for a prolonged time.

### Detailed Description of the Invention

Within the context of this specification the word "comprises" is taken to mean "includes, among other things". It is not intended to be construed as "consists only of".

Within the context of the present invention, the term "sufficient amounts" of substance is a parameter that depends strongly on the kind of substance that is used and of the individual that intends to consume the substance. Sufficient values for caffeine were discussed above. The skilled person is usually aware of the amount of an substance necessary to achieve a desired physiological effect.

In the context of the present invention, the term food product is intended to include any product that is consumed orally. It is, however, not intended to include products that are just chewed or dwindled or melted in the mouth, such as chewing gums, lollypops or sweets that are consumed in that way, for example. Further, the term is not intended to include pills, capsules or tablets that are just swallowed.

In the context of the present invention, the term "soluble fibre" means that fibre is at least 50% soluble according to the method of L. Prosky et al., J. Assoc, Off. Anal. Chem. 71, 1017-1023 (1988). Examples include inulin, pectin, β-glucan, gum arabic, tragacanth mucilages, guar and locust bean gum, agar, carageenans, alginates, xanthan and the like.

In an embodiment of the present invention, the soluble fibre is viscous soluble fibre. The viscous soluble fibre may be any suitable soluble fibre, which is able to increase the viscosity of the contents of the stomach and the small intestine. Examples are β-glucan, gums, such as guar gum, xanthan gum, and gum arabic, pectin, and, or mixtures of these. β-glucan is particularly preferred.

The food product according to the present invention, comprises, in percent by weight, 2 to 40% of viscous soluble fibre. Preferably, it comprises 3 to 30%, more preferably 5 to 20% of viscous soluble fibre. For example, the food product may comprise 7 to 16% viscous soluble fibre.

In a further embodiment, the food product according to the present invention comprises, in percent by weight of the raw ingredients, 20 to 80% oat bran concentrate. Preferably, it comprises 30 to 70%, more preferably 40 to 60% oat bran concentrate.

In still a further embodiment, the food product according to the present invention comprises, in percent by weight, 5 to 20% of protein, 1 to 10% of fats and/or oils and 40 to 70% of carbohydrates. Preferably, the product comprises 10 to 15% of protein, 2 to 7% of fats and/or oils, and 50 to 60% of carbohydrates.

The protein, fats and carbohydrates may be of any plant or animal origin. For example, the may substantially be comprised in cereals or other components used to manufacture the food product (see below), for example from oat bran concentrate.

In an embodiment of the use according to the present invention, the food product is suitable for athletes, sportsmen or physically active people.

In an embodiment of the consumable package according to the present invention, the solid product is a bar and the liquid product a beverage.

Soluble fibre may be derived from any natural source and is commercially available.

The (β-glucan may be derived from any source known to contribute β-glucan. In general, also fibre rich cereal may be used. Such a cereal could also be used as a source of carbohydrate, if desired. Preferably the fibre rich cereal contains at least about 6 to 10% by weight of (β-glucan and at least about 20% to 25% by weight of total fibre. The fibre rich cereal is preferably an oat bran concentrate or a barley flour; more preferably a de-fatted oat bran or barley flour.

In this specification, a "de-fatted oat bran concentrate" may be an oat bran fraction which has a (β-glucan content of above 10% by weight and which has been subjected to solvent extraction to remove, at least partially, oil and fats from the fraction. Ordinarily, oat bran concentrates have a fat or oil content of greater than about 5% by weight. De-fatted oat bran concentrates have an oil content of less than about 7% by weight; more usually about 4% to about 6% by weight.

De-fatted oat bran concentrates offer the advantage of better stability of the food product, easier processing, and improved texture and organoleptic properties of the food product.

De-fatted oat bran concentrates of this type are commercially available; for example suitable oat bran concentrates may be purchased from Swedish Protein AB, Väröbacka, Sweden. Alternatively the oat bran concentrate may be prepared by grinding dry oat grains and then carefully screening the fibre material from the starchy components of the oat grains. The fibre rich material may then be subjected to solvent extraction techniques to remove oils and fats from the material. A suitable procedure for the extraction of oils and fats is disclosed in British patent 1,526,553. The solvent extraction step may also carried out prior to screening if desired. This screening and extraction procedure would be suitable for producing oat bran concentrates with fibre contents at the lower end of the range; for example an oat bran concentrate having a maximum β-glucan content of about 15% by weight.

Caffeine can be obtained or used from a variety of sources. For example, extract of guarana, coffee, mate, black tea or green tea may be used.

The optimal quantities of caffeine have already been determined and the optimal concentrational range is known. It is recommended to not consume more than 1 gram per day and to ingest a dosage of 200mg to 400mg to be repeated no sooner than after three or four hours, as needed. The optimal plasma concentration of caffeine lies between 1.5 to 10mg/l, preferably between 2 to 6mg/l plasma. The threshold value of stimulation lies at about 2mg/l plasma.

There is no general rule as how the combination of a soluble fibre with the caffeine has to be achieved in order to obtain the food product according to the present invention.

It is possible to produce a food product, which may be liquid or solid, comprising the combination of soluble fibre and caffeine. Such a food product may be a solid or soft bar. The consumable product may also be in the form of a cookie, a biscuit, a snack, a cracker a bread or a bread like product. It is likewise possible to add the caffeine and soluble fibre to other food products, such as dairy products, ice cream, confectionery or beverages.

For example, sufficient amounts of caffeine may be added to whole grain oat flour, enriched with de-fatted oat bran to guarantee sufficient amount of soluble fibre. This mixture may be used to form a bread dough.

If the consumable product according to the present invention is intended to be a bar, there are a variety of possibilities to incorporate the caffeine and soluble fibre nce into a bar. In US 4,871,557 a method for obtaining a bar with supplemental dietary fibre is disclosed. The bar according to this reference is produced by extruding a fibre mixture containing apple fibre, corn bran, water and rice flour (the latter having the function of a binder), which is afterwards particle sized, i.e. shredded or ground to obtain flakes. These flakes high in fibre are then added to the other conventional ingredients of the bar. More concretely, the fibre mixture is added to other dry ingredients as grains (e.g. toasted rolled oats or crisped rice), nuts, fruits and/or chocolate chips. These dry ingredients are mixed with the extruded-shredded fibre-flakes and a (liquid) syrup blend is added. Other ingredients may be added and the mixture is transferred to a conventional bar forming line, which subjects the mixture to a forming, heating and a cutting procedure in order to obtain a bar.

This method may easily modified in order to obtain the food product according to the present invention. For example, (β-glucan may be added in the form of oat bran concentrate to the cereals prior to extrusion. The caffeine may be added at the same time or later. For example the caffeine may be added to the syrup.

A bar may also be prepared by mixing dry ingredients, which may be, besides soluble fibre, cereals, nuts, fruits, chocolate, berries, milk solids, for example. The dry ingredients of a bar may be later mixed with a binder, which is normally a syrup. The dry mixture will of a bar will usually comprise 40 to 90%, preferably 60 to 80% by weight of the total recipe, whereas the binder will account for the rest of it (60 to 30%, preferably 50 to 40% by weight).

Cereals may be just flour that is added to the dry mixture of the bar. However, cereals may be used in the form of crisps, flakes, puffs (oven or gun puffed), extruded and/or extruded-expanded cereals may serve as an example. Cereals are wheat, maize, barley, oat, rice, oat, millet and the like. Hence, cereals comprise, for example, rice or maize crisps, puffed rice or oat, any kind of flakes, baked or compressed and flaked cereals and so forth. Depending on the density of the final bar, the cereals may be chosen accordingly. For example, if a light bar is preferred, it is better to use crisps and/or puffs as cereals, whereas when a dense bar is preferred, it may be better to use flakes or baked and compressed cereals, or simply flour, such as rice flour, for example.

If high amounts of oat bran and/or oat bran concentrate (in order to provide β-glucan) are used to prepare a dry mixture for a bar, other cereals may be completely absent.

The dry mixture may also comprise nuts and fruits, for example. Examples are hazelnuts, wall-nuts, pecan-nuts, cashew nuts, almonds, coconut, chest nut, macadamia nut or mixtures of these. Nuts may be present in amounts up to 15%, preferably up to 10% by weight of the dry mixture. Fruits, such as apple, peach, pear, apricot, banana, orange, pine-apple, for example, and/or berries, such as raspberry, strawberry, blackberry, blueberry and the like may also be added to the dry mixture.

The binder to be added to the dry mixture may be syrup. A glucose syrup, for example, comprising a mixture of glucose and/or its polymers obtained by partial hydrolysis of starch, having a DE (dextrose equivalence) value of about 30 to 50 and a water content of from about 15 to 25% by weight of the binder. Generally, the binder may comprise invert sugar, glucose sugar and/or high fructose corn syrup, for example.

The binder may comprise milk solids, which is added in the form of milk powder and/or fresh milk. In the latter case, the addition of water may be at least partially replaced by the addition of milk. WO 0056171 describes a binder in this sense, which comprises 10 to 70 parts of sugar, 0.5 to 5 parts of a binding agent (a polysaccharide such as gum), up to 15 parts of glycerin, up to 60 parts of fruit pulp or cencentrate, up to 10 parts of cocoa powder and added water up to a water content of from 10 to 30%, for example.

The binder may comprise 0 to 15%, preferably 0.5 to 5% glycerol. Glycerol is sometimes added to bars to provide a moisture mouth-feel, while water content has generally to remain low to grant for a prolonged shelf life.

In a basic approach, the binder is simply a syrup comprising water (10 to 20% by weight of binder) and sugars, optionally further comprising fat (0 to 15%, preferably 2 to 10%), lecithin (0 to 1%, preferably 0.01 to 0.2%) and/or flavours, which is obtained by heating the water to 70 to 90°C and adding/dissolving the sugars under stirring. Syrups with a water content of about 15 to 20% by weight are also commercially available.

The caffeine may be added to the binder, or, alternatively, it may be added together with soluble fibre to the dry mixture, for example.

By mixing the dry mixture and the binder in the amounts mentioned above, a basic mass of the bar is obtained. This step may be done in any adequate mixing apparatus such as a screw mixer of the helical spring type with an axial sprinkling nozzle or with a coating drum, for example.

The basic mass may be transferred into a suitable forming or pressing apparatus, to shape the bar. For example, a Bepex-Hutt Roller Press type DP may be used, which pushes the basic mass through a slab nozzle or a strand nozzle, under pressures of up to 12 bar, preferably 5 to 10 bar. Another suitable apparatus is the Bepex-Hutt Roller Slab Former type GP, which does not imply such high pressures and therefore yields a flat paste with a lower specific weight.

However, the final bars may be obtained by other means, too. Generally, after forming the basic mass from the binder and the dry mixture, the further process may include forming rolling, pressure rolling, transfer on a pressure band, precooling (10 to 20°C), cutting of the final shape, for example, by a slitter or guillotine cutter (from Sollich or Rademaker), second cooling (4 to 15°C), and final packaging of the bar.

For example, if the cooked cereal product according to WO 9631128 is appropriately enriched with caffeine, this may be a sufficient method to obtain the food product according to the present invention.

The consumable package according to the present invention, comprising a liquid and a solid consumable product, wherein both components comprise caffeine may be obtained in any suitable manner.

For example, the solid consumable product may be a bar as described above, which is prepared comprising the necessary amount of soluble fibre. The liquid component may be a coffee or a tea for example.

Alternatively, the liquid product may be any beverage enriched with natural caffeine extracts derived from plants. For example, the liquid product may be a beverage comprising guarana powder, tea-or coffee extracts and the like.

In summary, the form of administration of the food product or the package according to the present invention is irrelevant, as long as soluble fibre together with caffeine is provided.

Without wishing to be bound by theory it is postulated that the soluble fibre binds to molecules and by this way delays its appearance in the blood stream. Another theory is that the soluble fibre creates a layer on the inner walls of the intestine that is able to prevent rapid absorption of caffeine.

The following examples are given by way of illustration only and in no way should be construed as limiting the subject matter of the present application. Percentages and parts are by weight unless otherwise indicated.

### Example 1: Manufacture of a bar comprising soluble fibre and caffeine

Three different kinds of bars are produced, wherein 200mg caffeine was added either in the form of a green tea extract or as guarana powder
OBC-GT: Oat bran cereal bar enriched with green tea extracts in the extrudate.
OBC-Gu: Oat bran cereal bar enriched with guarana in the extrudate.
OBC-GuS: Oat bran cereal bar enriched with guarana, added to the syrup.

The bars were prepared as follows.

First, expanded crisp were obtained by cooking-extrusion. In the bars OBC-GT and OBC-Gu, but not in OBC-GuS, the caffeine was added to the dry mixture, which was extruded and which further comprised about 78.0% oat bran concentrate, about 10.0% rice flour, about 9.0% wheat flour, about 2% powdered malt extract, and minor amounts of salts and colouring agents is prepared. The oat bran concentrate is obtained from Swedish Protein AB, Väröbacka, Sweden and contains about 35% dietary fiber in total and about 17% soluble fiber, substantially β-glucan.

The dry mixture is fed into a Clextral BC-45H extruder (screw diameter of 55 mm and a length of 600 mm) at a rate of about 79.6 kg/hr. Water is fed into the extruder at a rate of about 8.4 kg/hr. The extruder screws are rotated at 270 rpm. The temperature of the three zones of the extruder are 18°C, 69°C and 149°C. The temperature at the exit of the extruder is 184°C. The pressure in the extruder reaches 137 bar. The product leaving the extruder orifices is cut into pieces of length of about 2 to 3 mm and the pieces allowed to expand.

The pieces of expanded product are then air dried to a moisture content of about 1.6 % using air at about 120°C. The dried pieces have a crispy, pleasant texture and good mouthfeel. The β-glucan content is about 13 %.

Secondly, cereal flakes were prepared also by extrusion, that is by mixing about 97% oat bran concentrate (see above), about 2% powdered malt extract, and minor amounts of salts and coloring agents.

The dry mixture is fed into a Clextral BC-45H extruder as described above. The rate at which the mixture is fed is about 65.5 kg/hr. Water is fed into the extruder at a rate of about 16.2 kg/hr and an edible oil mixture is fed at a rate of 0.47 kg/hr. The extruder screws are rotated at 300 t-pm. The temperature of the three zones of the extruder are 18°C, 74°C and 103°C. The temperature at the exit of the extruder is 122°C. The pressure in the extruder reaches 126 bar. The product leaving the extruder orifices is cut into pieces of length of about 2 to 3 mm. The pieces are then flaked in a flaking apparatus (obtained from Buhler AG, Switzerland) and the flakes are then dried as the crisp balls above. The flakes have a water content of 2.3%. The dried flakes have a crispy, pleasant texture, a dietary fiber content of about 29 % and a soluble fiber content of about 14.5 %. The flakes have a good mouthfeel.

Thirdly, the bars are prepared, by mixing 13kg of the crisps (comprising caffeine in the case of bars OBC-GT and OBC-Gu) with 45kg of the flaked cereal product and 15kg of dried raisins. A fructose syrup is prepared from fructose, arabic gum and water. In the case of the bar OBC-GuS, caffeine was added to the syrup at the end and the syrup is combined with the crisp balls, the flakes and the raisins. The mixture is formed into bars, cooled and cut into 20g portions, all in a conventional manner.

The bars comprise each 200mg caffeine, they further have a β-glucan content of about 8% by weight, a protein content of about 12% by weight, a fat content of about 4.5% by weight, and a carbohydrate content of about 56% by weight.

### Example 2 : Comparison of caffeine absorption of caffeine in a bar comprising soluble fibre vs caffeine without soluble fibre.

### METHODS AND INDIVIDUALS

Eight male, healthy and non-smoking individuals of 20 - 40 years have been selected according to criteria that minimise variables interfering with the kinetics of caffeine (body weight and so forth).

Five products were tested:
1. Necafé Gold ® (NG)
2. Necafé Gold ® with white bread (62 g) (NG-WB)
3. Oat bran cereal bar enriched with green tea (OBC-GT)
4. Oat bran cereal bar enriched with guarana (OBC-Gu)
5. Oat bran cereal bar enriched with guarana, added to the syrup (OBC-GuS).

The products 3 - 5 correspond to the bars of Example 1.

All products comprise 200 mg caffeine.

Every individual was administrated all 5 products in a cross-over design, according to a randomisation plan of 7 treatments. The "wash-out" (after each treatment phase) was at least one weak. The products were consumed together with 150ml liquid (coffee or water) on empty stomach, without milk and sugar.

Blood samples were taken just before administration (time = 0) and then at the following times (minutes) after administration: 15, 30, 45, 60, 90, 120, 150, 180 (9 blood samples per individual). Salivary samples were taken for a prolonged period: 0, 15, 30, 45, 60, 90, 120, 150, 180, 210, 240, 300, 360, 480 minutes after administration.

For each individual and each product, the absorption rate of the caffeine has been determined by pharmacokinetical methods. The plasma and salivary kinetics of caffeine after administration was calculated and modelised according to a model (absorption of first order) according to the Bateman function (Bonai et al, 1982; Zylber-Katz et al, 1984). In fact, caffeine is a liposoluble molecule that is rapidly and completely absorbed at the gastro-intestinal level.

The pharmacokinetics were used to modelise caffeine plasma levels based on the data of the blood and salivary samples. Generally, plasma values are higher that salivary, however the constant ratio between them permits to reconstitute pharmacokinetics of plasma caffeine from 0 to 480 minutes by estimating plasma values beyond 180 minutes.

For each individual and each product, the model of the caffeine kinetics was calculated from the measured data. For each calculable model, the absorption speed with respect to the time (Tₘₐₓ) of highest concentration (Cₘₐₓ) was characterized. Furthermore, the elimination constant (Kel) and the surface of the concentration curves (AUC₍₀₋ₜ₎) of plasma and saliva was calculated.

### RESULTS

The results of the plasma samples are depicted in Figure 1. All products comprising caffeine and β-glucan show a slower absorption rate and usually reach a peak much later that the products comprising coffee or coffee and bread only. The maximal caffeine concentration (peak) is usually higher in products without OBC, and is also very quickly reached in these samples. In all products comprising oat bran the caffeine was absorbed slowly. In these samples, caffeine levels raised slowly and constantly, without abrupt change. After 3 hours, caffeine levels were usually higher in the samples were caffeine was consumed together with soluble fibre.

Figure 2 shows modelised curves based on the pharmacokinetics of the plasma and salivary samples. With the exception of the OBC-GuS sample, caffeine levels were higher 2 ½ hours to 8 hours after administration when caffein has been administrated together with soluble fibre. Higher values were maintained for a prolonged time if compared to caffeine consumed in the form of soluble coffee. Over the entire period from 30 minutes to 8 hours, caffeine levels remained more constant in all samples were caffeine and soluble fibre were consumed together.

### CONCLUSION

Food products comprising soluble fibre together with caffeine are capable of achieving sustained, prolonged, constantly relatively high values of caffeine in plasma and saliva. After prolonged time, values of caffeine remain high, even up to 8 hours. On the other hand, a high peak shortly after administration is avoided, thereby also avoiding side effects due to rapidly and very high concentrations of caffeine in blood. In general, concentration of the caffeine in blood or saliva is more constant and balanced over time. Best results are achieved when the caffeine is directly mixed with the soluble fibre when a food product is manufactured.

### Example 3: Consumable package consisting of a liquid and a solid product comprising caffeine

This example starts from the assumption that in specific circumstances it is wished to have instantly a high serum concentration of caffeine, and, in addition, that the high concentration be sustained for a prolonged time.

Hence, the blood concentration of caffeine is simulated by calculating the data of the consumption of a beverage comprising caffeine (soluble coffee) together with the data obtained from the consumption of the bar according to Example 1 (caffeine and soluble fibre). The bar and the coffee are selected to form an example of the consumable package according to the present invention.

The result can be derived from Figure 3. A high peak of caffeine is achieved quickly after consumption of the consumable package. The high serum concentration remains constantly high for more than three hours and then decreases slowly only.

Finally, for almost 6 hours, a threshold value of 2 mg caffeine /l serum is trespassed.

In conclusion, the consumable package consisting of a liquid and a solid component is apt to quickly cause a high serum concentration of caffeine and to sustain the high concentration for a prolonged time.

## Claims

1. Non-therapeutic use of a mixture of caffeine and soluble fibre in sufficient amounts in a food product to avoid short term peaks of the caffeine in plasma and/or to keep the plasma concentration of caffeine maintained for a prolonged period.

2. Non-therapeutic use of a mixture of caffeine and soluble fibre in sufficient amounts in a food product to slow down the absorption rate of the caffeine in the gastrointestinal tract.

3. Use of a mixture of caffeine and soluble fibre in sufficient amounts in the manufacture of a therapeutic food product to avoid undesired side effects of short term peaks of caffeine in plasma.

4. The use of Claim 1, 2 or 3, wherein the product contains 2 to 40% by weight of soluble fibre.

5. The use of any preceding claim, wherein the soluble fibre is viscous soluble fibre.

6. The use of Claim 5, wherein the viscous soluble fibre is β-glucan.

7. A food product comprising from 2 to 40% by weight of viscous soluble fibre and from 200 to 400 mg caffeine.

8. The food product of Claim 7, wherein the viscous soluble fibre is β-glucan.

9. The food product of Claim 7 or 8, further comprising, in percent by weight, 5 to 20% of protein, 1 to 10% of fats and/or oils, and 40 to 70% of carbohydrates.

10. Consumable package comprising a liquid and a solid product, wherein the solid product is the food product according to any of claims 7 to 9 and the liquid product also comprises caffeine.

11. Consumable package according to claim 10 wherein the solid product is a bar and the liquid product a beverage.

## Patentansprüche

1. Nicht-therapeutische Verwendung einer Mischung aus Coffein und einem löslichen Ballaststoff in ausreichenden Mengen in einem Lebensmittelprodukt, um kurzzeitige Spitzen des Coffeins im Plasma zu vermeiden und/oder um die Plasmakonzentration von Coffein über einen verlängerten Zeitraum aufrecht zu halten.

2. Nicht-therapeutische Verwendung einer Mischung von Coffein und einem löslichen Ballaststoff in ausreichenden Mengen in einem Lebensmittelprodukt, um die Absorptionsgeschwindigkeit des Coffeins im Magen-Darm-Trakt zu verlangsamen.

3. Verwendung einer Mischung von Coffein und einem löslichen Ballaststoff in ausreichenden Mengen bei der Herstellung eines therapeutischen Lebensmittelprodukts zur Vermeidung unerwünschter Nebenwirkungen von kurzzeitigen Spitzen des Coffeins im Plasma.

4. Verwendung nach Anspruch 1, 2 oder 3, wobei das Produkt 2 bis 40 Gew.-% löslichen Ballaststoff enthält.

5. Verwendung nach irgendeinem vorausgehenden Anspruch, wobei der lösliche Ballaststoff ein viskoser löslicher Ballaststoff ist.

6. Verwendung nach Anspruch 5, wobei der viskose lösliche Ballaststoff β-Glucan ist.

7. Lebensmittelprodukt, das von 2 bis 40 Gew.-% viskosen löslichen Ballaststoff und von 200 bis 400 mg Coffein umfasst.

8. Lebensmittelprodukt nach Anspruch 7, wobei der viskose lösliche Ballaststoff β-Glucan ist.

9. Lebensmittelprodukt nach Anspruch 7 oder 8, das außerdem, in Gew.-%, 5 bis 20 % Protein, 1 bis 10 % Fette und/oder Öle und 40 bis 70 % Kohlenhydrate umfasst.

10. Verzehrbare Fertigpackung, die ein flüssiges und ein festes Produkt aufweist, wobei das feste Produkt das Lebensmittelprodukt nach irgendeinem der Ansprüche 7 bis 9 ist und das flüssige Produkt ebenfalls Coffein umfasst.

11. Verzehrbare Fertigpackung nach Anspruch 10, wobei das feste Produkt ein Riegel ist und das flüssige Produkt ein Getränk ist.

## Revendications

1. Utilisation non thérapeutique d'un mélange de caféine et de fibre soluble en quantités suffisantes dans un produit alimentaire pour éviter les pics à court terme de la caféine dans le plasma et/ou pour stabiliser la concentration plasmatique de caféine pendant une période de temps prolongée.

2. Utilisation non thérapeutique d'un mélange de caféine et de fibre soluble en quantités suffisantes dans un produit alimentaire pour ralentir la vitesse d'absorption de la caféine dans le tractus gastro-intestinal.

3. Utilisation d'un mélange de caféine et de fibre soluble en quantités suffisantes dans la fabrication d'un produit alimentaire thérapeutique pour éviter les effets secondaires indésirables des pics à court terme de caféine dans le plasma.

4. Utilisation selon la revendication 1, 2 ou 3, dans laquelle le produit contient 2 à 40 % en poids de fibre soluble.

5. Utilisation selon l'une quelconque des revendications précédentes, dans laquelle la fibre soluble est une fibre soluble visqueuse.

6. Utilisation selon la revendication 5, dans laquelle la fibre soluble visqueuse est le β-glucane.

7. Produit alimentaire comprenant de 2 à 40 % en poids de fibre soluble visqueuse et de 200 à 400 mg de caféine.

8. Produit alimentaire selon la revendication 7, dans lequel la fibre soluble visqueuse est le β-glucane.

9. Produit alimentaire selon la revendication 7 ou 8, comprenant en outre, en pourcentage en poids, 5 à 20 % de protéines, 1 à 10 % de matières grasses et/ou d'huiles, et 40 à 70 % de glucides.

10. Emballage consommable comprenant un produit liquide et un produit solide, dans lequel le produit solide est le produit alimentaire selon l'une quelconque des revendications 7 à 9 et le produit liquide comprend également de la caféine.

11. Emballage consommable selon la revendication 10, dans lequel le produit solide est une barre et le produit liquide une boisson.
